# EUROPEAN PATENT APPLICATION

(11) **EP 3 845 117 A1**
(43) Date of publication of application: **07.07.2021**
(21) Application number: 19854380.3
(22) Date of filing: 08.08.2019
(51) Int. Cl.: A61B 1/04, A61B 1/00

(54) **ELECTRONIC ENDOSCOPE AND ELECTRONIC ENDOSCOPE SYSTEM**

(30) Priority: 31.08.2018 CN 201811011600
(71) Applicant: Shanghai Microport Medbot (Group) Co., Ltd., Shanghai 201203 (CN)
(72) Inventor: MAO, Haoyang, Shanghai 200135 (CN); HE, Yuyuan, Shanghai 200135 (CN); HE, Chao, Shanghai 200135 (CN); GAO, Xu, Shanghai 200135 (CN); QU, Min, Shanghai 200135 (CN); WANG, Yan, Shanghai 200135 (CN)
(74) Representative: Roberts, Peter David
(86) International application number: PCT/CN2019/099866
(87) International publication number: WO 2020/042887

(57) **Abstract**

An electronic endoscope and an electronic endoscope system, wherein the electronic endoscope comprises a tube body (100), at least two lenses (200) having a non-zero-degree viewing angle, an image sensing component (300), and a transmission module. The tube body (100) comprises a light receiving channel penetrating therein for sequentially accommodating the lenses (200), the image sensing component (300), and the transmission module. Optical axes of the lenses (200) are parallel to an extension axis of the light receiving channel. The image sensing component (300) receives images transmitted by the lenses (200) and converts the same into electrical signals. The transmission module drives the lenses (200) having a non-zero-degree viewing angle to rotate about the optical axes in the same direction. When the field-of-view region of the electronic endoscope changes, the field-of-view direction does not change.

## Description

### CROSS-REFERENCE TO RELATED APPLICATION

This application claims priority to Chinese patent application No. 201811011600.2 and titled "Electronic endoscope and electronic endoscope system", filed on August 31, 2018, the entire contents of which are incorporated herein by reference in its entirety for all purposes.

### TECHNICAL FIELD

The present disclosure relates to the technical field of medical device, in particular to an electronic endoscope and an electronic endoscope system.

### BACKGROUND

With the rapid development of interventional medical technology, an electronic endoscope, which integrates traditional optical endoscopy technology, modern computer technology, microelectronics technology and other high-tech technologies, has become a very widely used medical diagnostic instrument.

In the use of electronic endoscope, due to the small incision of minimally invasive operation, it is usually necessary to rotate the angle of endoscope to observe the target tissue or organ. However, for electronic endoscopes, no matter 2D or 3D electronic endoscopes, when the operator rotates the lens body, the lens and the photosensitive element rotate simultaneously, which results in that the field of view (FOV) direction will rotate with the change of the field of view area, such that it is inconvenient for the doctor to diagnose.

### SUMMARY OF THE INVENTION

An electronic endoscope and an electronic endoscope system are provided according to various embodiments of the present disclosure.

In one aspect of the present disclosure, an electronic endoscope is provided. The electronic endoscope includes a tube body, at least two non-zero degree lenses, an image sensing assembly and a transmission module. The tube body is provided with a light receiving channel therethrough, which is configured to accommodate the lenses, the image sensing assembly, and the transmission module sequentially. Optical axes of the lens are parallel to an extension axis of the light receiving channel. The image sensing assembly is configured to convert the received image transmitted from the lenses into an electrical signal and output the electrical signal, and the transmission module is configured to drive the lenses to rotate about the optical axes in the same direction.

The tube body of the electronic endoscope mentioned above is provided with a light receiving channel therethrough. The optical receiving channel accommodates a lens, an image sensing assembly, and a transmission module sequentially. The optical axis of the lens is parallel to the extension axis of the light receiving channel. The image sensing assembly is configured to receive the image transmitted by the lenses and convert the image into an electrical signal. The transmission module is configured to drive each non-zero degree lens to rotate about its own optical axis in the same direction, such that each lens can rotate independently from the image sensing assembly, and the field of view direction will not change while the field of view area changes.

In one embodiment, the electronic endoscope also includes a driving device, and the transmission module includes a first transmission device, a second transmission device, and a transmission medium. The driving device drives the transmission medium via the second transmission device, and then the transmission medium drives the non-zero degree lenses via the first transmission device.

In one embodiment, the tube body includes a lens body sealing head and a main body of the tube body, wherein the lens body sealing head is connected to the distal end of the main body of the tube body. The light receiving channel includes a lens inner cavity located at the distal end of the lens body sealing head, a first light receiving channel located at the proximal end of the lens body sealing head, and a second light receiving channel extending through the main body of the tube body, wherein the lens extends from the lens inner cavity to the first light receiving channel, the first transmission device is located in the first light receiving channel, and the image sensing assembly is located at the proximal end of the lens body sealing head and fixed in the second light receiving channel.

In one embodiment, the electronic endoscope also includes a handheld end located at the proximal end of the tube body. The driving device is located on the handheld end. The second transmission device is located at the proximal end of the light receiving channel and is connected to the driving device. And the proximal end of the transmission medium is connected to the second transmission device, and extends to a distal end along the axial direction of the tube body and is connected to the first transmission device.

In one embodiment, the first transmission device includes any one of a gear transmission mechanism, a belt transmission mechanism, a chain transmission mechanism, a connecting rod transmission mechanism or a wire transmission mechanism. The transmission medium is a transmission shaft or a steel wire. The second transmission device includes a gear transmission mechanism or a connecting shaft structure.

In one embodiment, the image sensing assembly includes a printed circuit board and at least one solid camera element provided on the printed circuit board, the fixed camera element has a photosensitive surface, wherein the imaging surface of the lens is located on the photosensitive surface of the solid camera element.

In one embodiment, the printed circuit board and the photosensitive surface of the solid camera element are perpendicular to the optical axis, a through hole is formed on the printed circuit board, wherein the first transmission device passes through the through hole.

In one embodiment, the first transmission device includes a gear transmission mechanism. The gear transmission mechanism includes a driving gear and at least two lens gears. Each of the lens gears is coupled to an outside of one lens and is engaged with the driving gear. The driving gear is connected to the transmission medium. The driving gear drives each lens gear rotate in the same direction, such that each lens rotates in the same direction.

In one embodiment, the printed circuit board and the photosensitive surface of the solid camera element are arranged along the direction of the optical axis. The electronic endoscope further includes an optical turning component, and the optical turning component is configured to convert a propagation direction of an outgoing light of the lens into a direction perpendicular to the photosensitive surface.

In one embodiment, the first transmission device includes a gear transmission mechanism. The gear transmission mechanism includes a gear mounting base, a driving gear, a driven gear and at least two lens gears. Each lens gear is coupled to an outside of one lens. The driving gear is connected to the transmission medium. The driving gear and the driven gear can be both rotatably fixed on the gear mounting base. The driving gear is externally engaged with the driven gear, and the driven gear is externally engaged with each lens gear to drive each lens gear to rotate in the same direction.

In one embodiment, the optical turning component is a turning prism or a plane mirror.

In one embodiment, the outgoing surface of the turning prism is attached to the photosensitive surface of the image sensing assembly.

In one embodiment, a channel is formed on the printed circuit board allowing the transmission medium to pass through.

In one embodiment, the printed circuit board includes at least two sub printed circuit boards, each of which is symmetrically arranged with respect to the extension axis of the tube body to form a channel allowing the transmission medium to pass through.

In one embodiment, the electronic endoscope further includes a lens shield provided at a distal end of each lens inner cavity, and the lens shield is configured to protect the lens in the corresponding lens inner cavity.

In one embodiment, the electronic endoscope further includes a sealing shield which is provided on the lens body sealing head.

In one embodiment, the sealing shield is connected to the lens body sealing head via a snap-fit connection or a thread connection.

In one embodiment, the electronic endoscope further includes an optical fiber. The lens body sealing head forms an optical output channel therethrough between the lens inner cavities. The optical output channel is in communication with the second optical receiving channel, and the optical fiber extends from the second optical receiving channel and extends to the distal end of the lens body sealing head through the optical output channel.

In one embodiment, the electronic endoscope further includes a cold light source and a power supply, wherein the lens body sealing head forms a light output channel between the lens inner cavities, and the light output channel is configured to accommodate the cold light source and the power supply.

Another aspect of the present disclosure provides an electronic endoscope system, which includes an electronic endoscope as described in any of the preceding embodiments, an image workstation connected to the image sensing assembly and configured to receive the image information transmitted by the image sensing assembly and processing the image information, and a monitor connected to the image workstation for receiving and displaying the image information processed by the graphic workstation.

### BRIEF DESCRIPTION OF THE DRAWINGS

FIG. 1 is a perspective view of an electronic endoscope according to an embodiment;
FIG. 2a is a sectional view of an electronic endoscope in the embodiment shown in FIG. 1;
FIG. 2b is a partial enlarged view of FIG. 2a;
FIG. 2c is an exploded view of the portion shown in FIG. 2b;
FIG. 3 is a schematic view of the transverse section structure of the first transmission device in the embodiment shown in FIG. 2b;
FIG. 4 is a schematic view of the transverse section structure of the first transmission device according to an embodiment;
FIG. 5 is a schematic view of the transverse section structure of the first transmission device according to an embodiment;
FIG. 6 is a schematic view of the transverse section structure of the first transmission device according to an embodiment;
FIG. 7 is a local structure view of an electronic endoscope with a driving device according to an embodiment;
FIG. 8 is a local structure view of an electronic endoscope having a driving device according to an embodiment;
FIG. 9 to FIG. 12 are schematic views of the electronic endoscope in the embodiment shown in FIG. 2 in different postures;
FIG. 13 is a structural view of the sealing cover of an electronic endoscope according to an embodiment;
FIG. 14 is a partial exploded view of an electronic endoscope according to an embodiment;
FIG. 15 is a schematic view of the transverse section structure of the first transmission device in the embodiment shown in FIG. 14;
FIG. 16 to FIG. 19 are schematic views of the electronic endoscope in the embodiment shown in FIG. 14 under different postures;
FIG. 20 is a schematic view of an electronic endoscope system according to an embodiment.

### DETAILED DESCRIPTION

In order to make the purpose, technical scheme and advantages of the application clearer and clearer, the present application is further described in detail in combination with the attached drawings and embodiments. It should be understood that the specific embodiments described herein are only for the purpose of explaining the present application and are not intended to limit the present application.

In the description of the present application, it should be understood that the orientation or position relationship indicated by the terms "center", "horizontal", "up", "down", "inside" and "outside" are based on the orientation or position relationship shown in the drawings, which is only for the convenience of describing the application and simplifying the description, rather than indicating or implying that the device or element referred to must have a specific orientation and special features. Therefore, it cannot be understood as a limitation of the present application. In addition, it should be noted that when an element is considered to be "connected" to another element, it can be directly connected to the other element or there are intermediate elements simultaneously. Conversely, when an element is referred as "directly on" another element, there is no intermediate element. In the present application, the end close to the patient is the distal end or the far end, and the end far away from the patient is the proximal end or front end.

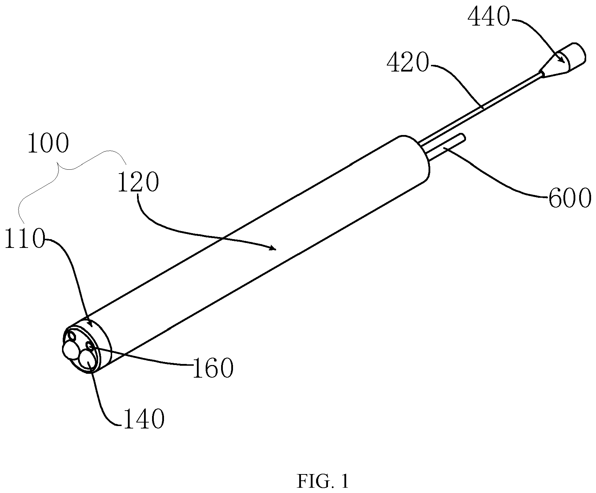

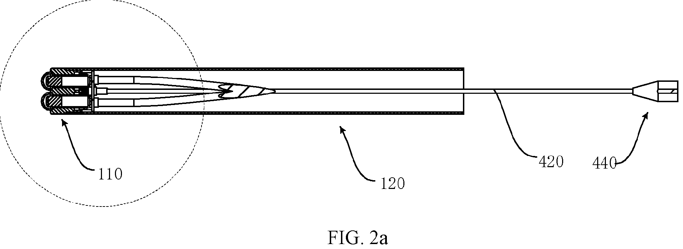

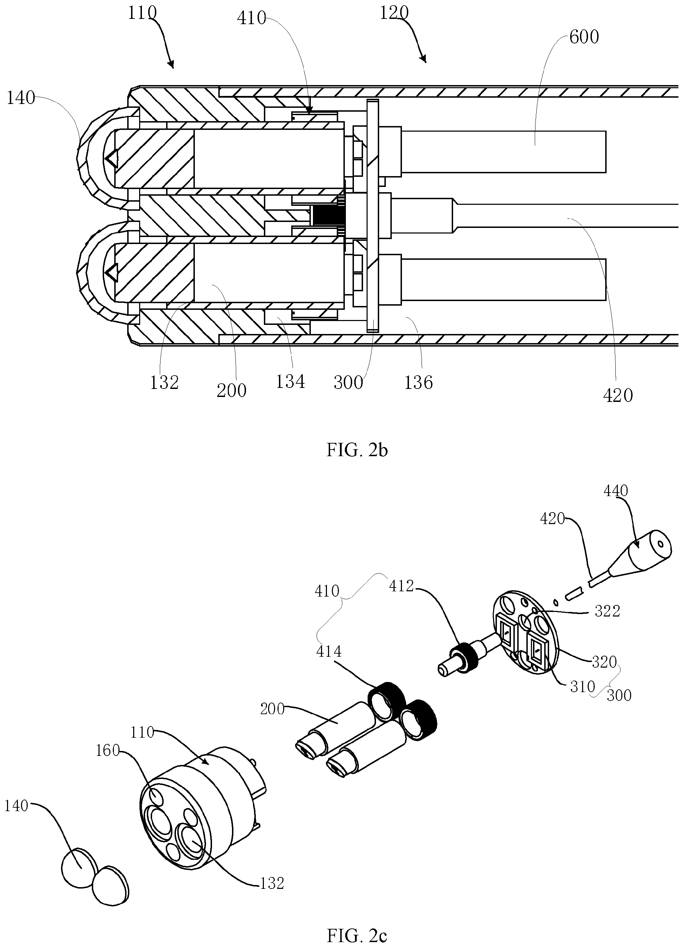

FIG. 1 is a perspective view of an electronic endoscope according to an embodiment. FIG. 2a is a sectional view of the electronic endoscope in the embodiment shown in FIG. 1. FIG. 2b is a partial enlarged view of FIG. 2a. FIG. 2c is an exploded view of the portion shown in FIG. 2b. The structure of the electronic endoscope is described in detail in combination with FIG. 1 and FIG. 2a to FIG. 2c.

In this embodiment, the electronic endoscope includes a tube body 100, at least two non-zero degree lenses 200, an image sensing assembly 300, and a transmission module. The tube body 100 is provided with a light receiving channel therethrough, which is configured to accommodate the at least two non-zero lenses 200 (hereinafter referred to as lenses), the image sensing assembly 300, and the transmission module. Specifically, the optical axes of the lenses 200 are parallel to an extension axis of the light receiving channel. In the present embodiment, the non-zero degree lens 200 refers to a lens with a non-zero viewing angle. For example, the viewing angle of the lens 200 is 30 degrees. In other embodiment, the viewing angle of the lens can also be other angles. The image sensing assembly 300 is configured to receive the image transmitted by the lens 200 and convert the image into an electrical signal for outputting, for example, to an image processing device or an image workstation. The transmission module is configured to drive each lens 200 to rotate about its own optical axis in the same direction, such that each lens 200 can rotate independently from the image sensing assembly 300. Moreover, when the FOV (field of view) area are changed, the FOV direction will not be changed, such that it will not bring any inconvenience to the doctor's diagnosis work. In each exemplary embodiment of the present disclosure, the field of view area refers to the visible area of the observed scene obtained through the lens 200. The direction of the field of view refers to the direction of the observed scene, which is obtained through the lens 200, presented on the image sensor assembly 300. For example, when the image sensing assembly 300 is placed upright, the direction of the observed scene on the image sensing assembly 300 is also a positive direction, that is, the FOV direction is a positive direction. When the image sensing assembly 300 rotates, the direction of the observed scene on the image sensing assembly 300 also deflects, that is, the FOV direction changes accordingly.

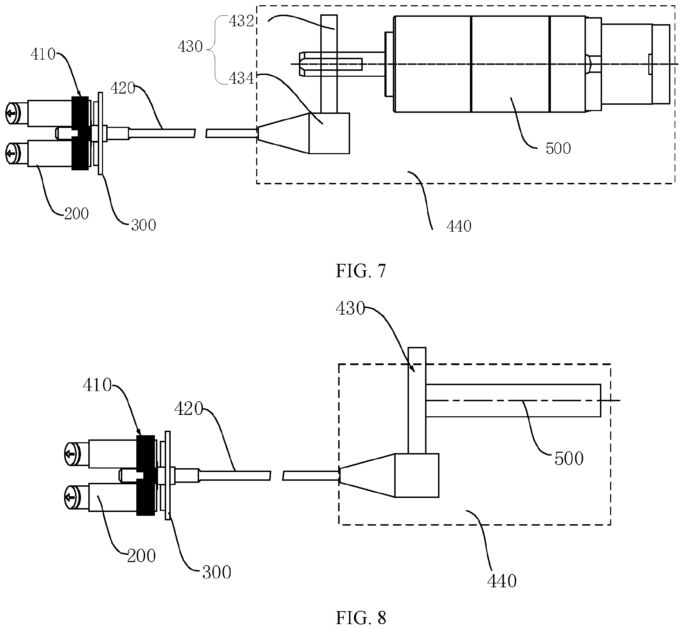

In an embodiment, the aforementioned electronic endoscope further includes a driving device 500, as shown in FIG. 7 and FIG. 8. In this embodiment, the transmission module includes a first transmission device 410, a second transmission device 430, and a transmission medium 420. The driving device 500 drives the transmission medium 420 via the second transmission device 430, and the transmission medium 420 drives each lens 200 via the first transmission device 410, such that each lens 200 rotates about its own optical axis in the same direction. In one specific embodiment, the driving device 500 is a driving motor, as shown in FIG. 7. The driving motor drives the transmission medium 420 via the second transmission device 430, and the transmission medium 420 drives each lens 200 to rotate about its own optical axis in the same direction via the first transmission device 410. In another specific embodiment, the driving device 500 can be a driving rod, as shown in the FIG. 8. In this embodiment, the driving rod drives the transmission medium 420 via the second transmission device 430, and then the transmission medium 420 drives each lens 200 to rotate about its own optical axis in the same direction via the first transmission device 410.

In an embodiment, the electronic endoscope further includes a handheld end 440 (shown in the dash line area of FIG. 7 and FIG. 8) located at the proximal end of the tube body 100. The driving device 500 is located inside or partially outside of the handheld terminal 440, so as to facilitate the operation for the doctors or operators. The second transmission device 430 is located in the handheld end 440. Further, the second transmission device 430 is adjacent to the proximal end side of the optical receiving channel and is connected to the driving device 500. The proximal end of the transmission medium 420 is connected to the second transmission device 430, the transmission medium 420 extends along the axial direction of the tube body 100, and the distal end thereof is connected to the first transmission device 410.

In an embodiment, the tube body 100 includes a lens body sealing head 110 and a main body of the tube body 120, as shown in FIG. 1, FIG. 2a and FIG. 2b. The lens body sealing head 110 is connected to the distal end of the main body of the tube body 120. Specifically, the optical receiving channel includes a lens inner cavity 132 located at the distal end of the lens body sealing head 110, a first light receiving channel 134 located at the proximal end of the lens body sealing head 110, and a second light receiving channel 136 through the main body of the tube body 120, as shown in FIG. 2b. The lens inner cavity 132, the first optical receiving channel 134, and the second optical receiving channel 136 are in communication with each other. The number of lens inner cavities 132 is the same as the number of lenses 200, such that each lens inner cavity 132 accommodates one lens 200. Therefore, the lens inner cavity 132 has a cavity structure matching the size of the lens 200. Specifically, each lens 200 extends into the first light receiving channel 134 from the corresponding lens inner cavity 132. In addition, the optical axis of each lens 200 is collinear with the extension axis of the corresponding lens inner cavity 132, and the extension axis of each lens inner cavity 132 is parallel to the extension axis of the tube body 100, such that each lens 200 has the maximum visible range. The first transmission device 410 is located in the first optical receiving channel 134, while the image sensing assembly 300 is located at the proximal end of the lens body sealing head 110 and is fixed in the second optical receiving channel 136.

In an embodiment, the first transmission device 410 is mainly configured to drive each lens 200 to rotate about its own optical axis in the same direction. The first transmission device 410 may include any one of a gear transmission mechanism, a belt transmission mechanism, a chain transmission mechanism, a connecting rod transmission mechanism and a wire transmission mechanism. Further, when the first transmission device 410 is a gear transmission mechanism, the engaging mode of the gear transmission mechanism can be an internal engagement between gears or an external engagement between gears, and is not limited to a specific implementation mode. The transmission medium 420 may be a transmission shaft or a steel wire. For example, the transmission shaft may be a rigid shaft or a flexible shaft. As for the steel wire, the transmission of torque is realized by driving coaxial bevel gear and turbine through a rotating wheel. There is no specially limitation on the second transmission device 430, it may be an externally engaged gear set, that is, the gear set includes a first gear 432 and a second gear 434, as shown in FIG. 7. The first gear 432 is provided on the output shaft of the driving motor, while the second gear 434 is located at the proximal end of the transmission medium 420. In other embodiments, the second transmission device 430 may also be a coupling or a universal joint. The connection between the output shaft of the driving motor and the transmission medium 420 can be realized by the universal joint or coupling, such that the control of rotation movement of the driving motor to each lens 200 can be realized.

In an embodiment, the image sensing assembly 300 includes at least one solid camera element 310 and a PCB (printed circuit board) 320. The solid camera element 310 is fixed on the PCB 320. The number of the solid camera elements 310 and the number of the lenses 200 may be the same or different. For example, all lenses 200 can correspond to one solid camera element 310. In an embodiment, the PCB 320 is fixedly arranged with respect to the lens body sealing head 110, that is, the solid camera element 310 is also fixedly arranged with respect to the lens body sealing head 110. The solid camera element 310 is configured to receive the image transmitted by the lens 200 and convert the image into an electrical signal. The position of the solid camera element 310 can be set as required, but it is necessary to ensure that a distance of the light from the outgoing surface of the lens 200 to the photosensitive surface of the solid camera element 3120 is equal to the image distance of the lens 200, that is, the imaging surface of the lens 200 is located at the photosensitive surface of the solid camera element 310, such that it can ensure that the lens 200 can converge the light reflected from human tissues, which can image on the photosensitive surface of the solid camera element 310. In one embodiment, the solid camera element 310 may be a charge coupled device (CCD) or a complementary metal oxide semiconductor (CMOS) commonly used in the art. After the image of the photographed object is converted into an electrical signal by the solid camera element 310, the electrical signal can be output to an image processing device, such as an image workstation 920, by the PCB 320, and after image processing (such as denoising, white balance, sharpening, etc.), the electrical signal can be transmitted to display devices such as a monitor 930, such that the medical staff can observe images on the monitor.

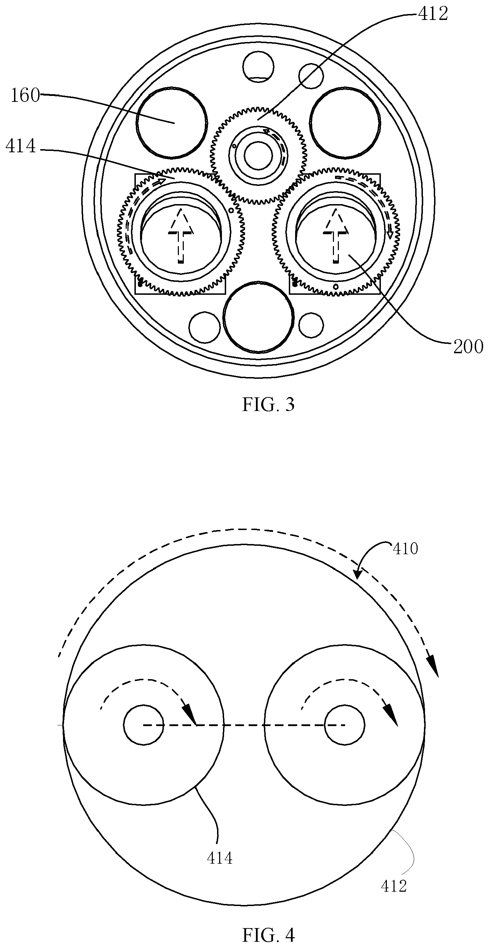

In an embodiment, the solid camera element 310 is mounted on the PCB 320, and the photosensitive surface of the solid camera element 310 is configured to face the outgoing surface of the lens 200, as shown in FIG. 2c. In other words, the solid camera element 310 and the PCB 320 are arranged along an optical axis direction of the lens 200. In this embodiment, the first transmission device 410 is a gear transmission mechanism. Specifically, the first transmission device 410 includes a driving gear 412 and at least two lens gears 414. The rotation axes of the driving gear 412 and the lens gear 414 are parallel to the optical axis of the lens 200, preferably collinear, as shown in FIG. 3. The number of the lens gears 414 corresponds to the number of the lenses 200 in the electronic endoscope. Each lens gear 414 is provided in the first light receiving channel 134. Each lens gear 414 is coupled to an outside of the corresponding lens 200 and is engaged with the driving gear 412. The driving gear 412 is also provided in the first receiving channel 134 and connected to the transmission medium 420. Therefore, the driving device 500 drives the transmission medium 420 via the second transmission device 430, and the transmission medium 420 drives each lens gear 414 to rotate about the optical axis of the corresponding lens 200 in the same direction via the driving gear 412, such that each lens 200 rotates about its own optical axis in the same direction. Further, the center of the drive gear 412 is located on the center perpendicular line of the line connecting the centers of the lens gears 414, that is, the distances from the center of the driving gear 412 to the centers of each lens gears 414 are the same.

In one embodiment, the PCB 320 is provided with a through hole 322 to allow the first transmission device 410 to extend through. Specifically, the rotating shaft of the driving gear 412 of the first transmission device 410 extends through the through hole 322 into the second light receiving channel 136 and is connected to the distal end of the transmission medium 320. At this time, the driving device 500 drives the transmission medium 420 via the second transmission device 430. The transmission medium 420 drives the driving gear 412 to rotate via the first transmission device 410, and then drives each lens gear 414 to put each lens 200 in rotation. Since the first transmission device 410 extends through the through hole 322 on the PCB 320, such that the first transmission device 410 can rotate with respect to the PCB 320. Therefore, in the process of realization of driving the rotation of the lens 200, the rotation of the first transmission device 410 will not drive the PCB 320 to rotate, so as to ensure that the PCB 212 and the solid camera element 310 fixed thereon can be fixed with respect to the lens body sealing head 110, without synchronous rotating with the lens 200. The relative independence of the rotation process of the both is realized, and then the FOV direction will not be changed while expanding the FOV range, which is convenient for the doctor's diagnosis work.

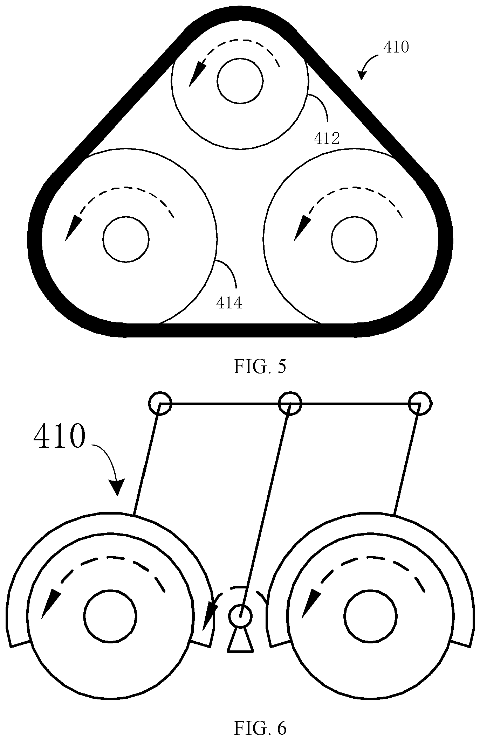

In an alternative embodiment, the driving gear 412 and the lens gear 414 of the first transmission device 410 may also be internally engaged, as shown in FIG. 4. At this time, the diameter of the driving gear 412 is at least greater than twice of the diameter of the lens gear 414. Further, the first transmission device 410 may be other structures. It should be understood by those skilled in the art that the first transmission device 410 of the present disclosure can be any device that can convert the torque transmitted by the transmission medium 420 into the driving force to drive the independent rotary motion of each lens 200. For example, the first transmission device 410 may also adopt a belt transmission mechanism (as shown in FIG. 5), a chain transmission mechanism, a link transmission mechanism (as shown in FIG. 6), or a wire transmission mechanism, etc. The dashed arrows in FIG. 4 to FIG. 6 demonstratively show the relationship of the directions of motion between the various components in the first transmission device 410, which does not constitute a limitation on the rotational ability of various components. The specific structure of the first transmission device 410 can be configured according to the requirement, for example, according to the number of lenses 200, the structure of the distal end of the endoscope, the limitation of the outer diameter, etc.

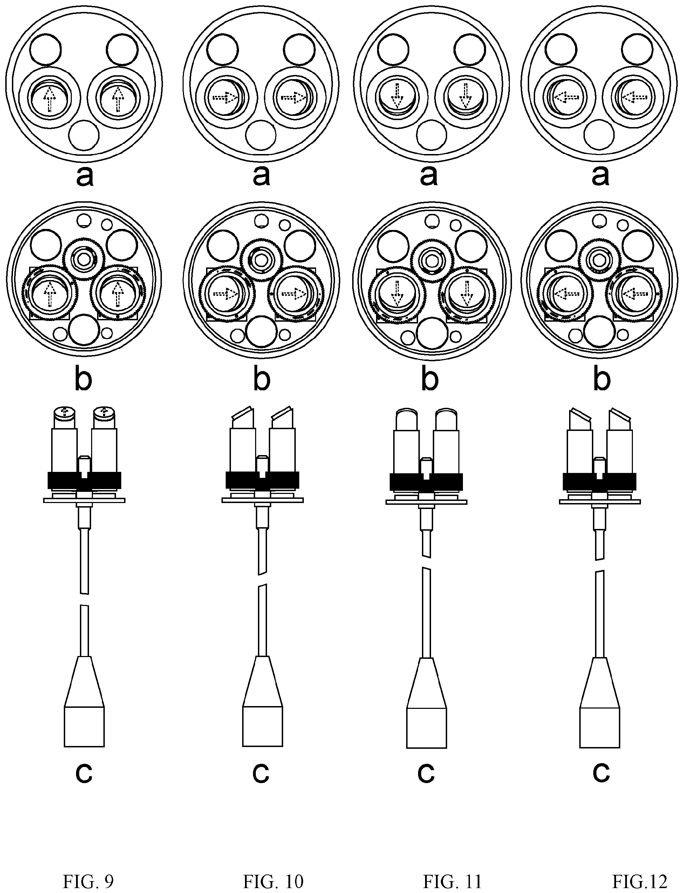

FIG. 9 to FIG. 12 are the schematic views of the electronic endoscope in the embodiment shown in FIG. 2 at four different postures of 0°, 90°, 180° and 270°, respectively, during the process of clockwise rotation with 360 degrees. The dashed arrow is used to identify the rotation direction of lens 200. It should be understood that the dashed arrows are for the convenience of explanation only and do not constitute a limitation to the present application. In the figures, *a* shows a schematic state of lens 200, *b* shows a schematic state of the first transmission device 410 corresponding to the state of *a,* and c shows the structural views of various components in the light receiving channel of the electronic endoscope in the state of *a.* In the present embodiment, two lens inner cavities 132 are provided in the lens body sealing head 110, that is, the electronic endoscope includes two lenses 200. It can be seen from FIG. 9 to FIG. 12 that the driving gear 412 is driven to rotate anticlockwise, meanwhile the two lens gears 414 are driven to rotate clockwise, such that the two lenses 200 are driven to rotate in the same direction simultaneously, while the PCB 320 and the solid camera element 310 will not rotate. Then it is achieved that the FOV direction will not be changed when expanding the visual line.

In one embodiment, the electronic endoscope further includes a lens shield 140 provided at the distal end of each lens inner cavity 132, as shown in FIG. 1 and FIG. 2a to FIG. 2c. The lens shield 140 is configured to protect the lens 200 and other components in the corresponding lens inner cavity 132, especially to maintain the lens 200 clean and prevent the lens 200 from being contaminated by tissue fluid and other pollutants during the operation. The lens shield 140 is fixed at the front end of the lens 200, and is fixed on the lens body sealing head 110 by means of a thread or a buckle, or is integrally formed with the lens body sealing head 110. The lens shield 140 can be made of a material with higher transparency, such that it will not affect the image acquisition.

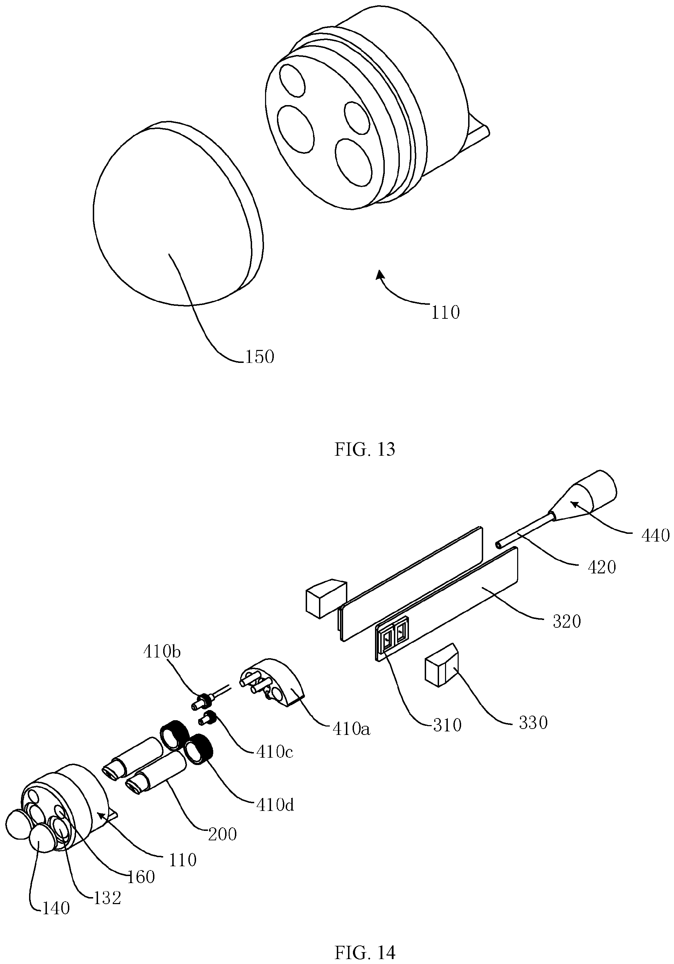

In another alternative embodiment, the lens shield 140 can be replaced by an integral sealing shield 150, as shown in FIG. 13. At this time, the sealing shield 150 is provided at the front end of the lens body sealing head 110 to protect the lens 200 and other components. The sealing shield 150 may be fixed on the lens body sealing head 110 by means of a snap-fit connection or a threaded connection.

In an embodiment, the lens body sealing head 110 is provided with a light output channel 160 therethrough between the lens inner cavities 132. The optical output channel 160 is communicated with the second optical receiving channel 136 as shown in FIG. 2b and FIG. 2c. Specifically, the number of the optical output channels 160 may be the same as or different from the number of the lens inner cavities 132. For example, in the present embodiment, there are three optical output channels 160 and two lens inner cavities 132. In this embodiment, the electronic endoscope further includes an optical fiber 600. The optical fiber 600 extends from the second optical receiving channel 136 and extends to the distal end of the lens body sealing head 110 through the optical output channel 160. The proximal end of the optical fiber 600 is connected to a separately arranged lighting device to output the light generated by the lighting device to the light output channel 160. In another embodiment, the electronic endoscope may further include a cold light source and a power supply (not shown in the figures). The cold light source and power supply are arranged in the light output channel. The power supply is configured to provide electrical energy to the cold light source, to enable the cold light source work as a lighting device to provide a light source to the light output channel 160. Since the cold light source is directly provided in the optical output channel 160, there is no need to provide an optical fiber in the second optical receiving channel 136. The volume of the electronic endoscope system can be reduced by arranging the cold light source and power supply in the lens body sealing head 110.

FIG. 14 is a partial exploded view of the electronic endoscope in another embodiment, in which the related components in the light receiving channel are mainly presented. In this embodiment, the components appearing in the above-mentioned embodiments continue to use the same reference symbols as those in the above-mentioned embodiments, and the components without special description are the same as those in the above-mentioned embodiment.

In this embodiment, the photosensitive surface of the solid camera element 310 and the PCB 320 in the image sensing assembly 300 are arranged along the extension axial direction of the tube body 100, that is, along the optical axis direction of the lens 200. Correspondingly, the electronic endoscope further includes an optical turning member 330. The optical turning member 330 is configured to convert the propagation direction of the outgoing light of the lens 200 into a direction perpendicular to the photosensitive surface of the solid camera element 310. Specifically, the number of optical turning members 330 corresponds to the number of lenses 200. Further, the optical turning member 330 can be a turning prism to achieve the turning of the incident light. Specifically, the turning prism can be a triple prism or a pentaprism. In addition, the optical turning member 330 may also be a planar mirror. An outgoing surface of the optical turning member 330 is located on the photosensitive surface of the solid camera element 310. Furthermore, the outgoing surface of the optical turning member 330 is attached to the photosensitive surface of the solid camera element 310, which can reduce the occupied space and is conducive to the miniaturization of the device. The other side of the solid camera element 310 is attached to the PCB 320. The position of the PCB 320 should be configured such that the distance from the light outgoing surface of the lens 200 via the optical turning member 330 to the photosensitive surface of the solid camera element 310 is equal to the image distance of the lens 200, such that it ensures that the light is imaged on the light sensitive surface of the solid camera element 310 after passing through the lens 200 and the optical turning member 330.

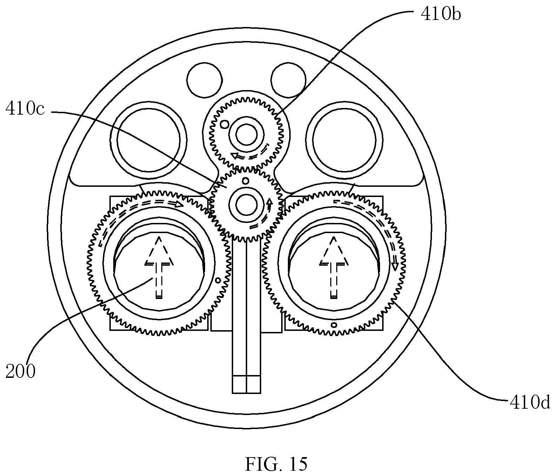

In this embodiment, the first transmission device 410 can also be one of a gear transmission mechanism, a belt transmission mechanism, a chain transmission mechanism, a connecting rod transmission mechanism or a wire transmission mechanism. For example, the first transmission device 410 is the gear transmission mechanism, as shown in FIG. 14 and FIG. 15. Specifically, the first transmission device 410 includes a gear mounting base 410a, a driving gear 410b, a driven gear 410c and at least two lens gears 410d. Each lens gear 410d is coupled to the outside of one lens 200. The driving gear 410b and the driven gear 410c can be rotatably fixed on the gear mounting base 410a. The driving gear 410b is connected to the transmission medium 420. The driving gear 410b is externally engaged with the driven gear 410c. The driven gear 410c is externally engaged with each lens gear 410d to drive each lens gear 410d to rotate in the same direction. Further, the distances from the center of the driven gear 410c to the center of each lens gear 410d remain the same.

Further, the PCB 320 has a hollow polygon structure, which can form a channel for accommodating the transmission medium 420. Therefore, the transmission medium 420 can transmit the input driving force to each lens 200 through the first transmission device 410, and then drive each lens 200 to rotate about its own optical axis in the same direction via the first transmission device 410. In another alternative embodiment, the PCB 320 includes at least two sub-PCBs, as shown in FIG. 14. At this time, the numbers of sub-PCBs, optical turning members 330, and lenses 200 are the same and in one-to-one correspondence. Each sub-PCB is symmetrically arranged along the axial direction of the tube body 100. In this embodiment, the PCB 320 includes two sub-PCBs. The two sub-PCBs are symmetrically arranged with respect to the extension axis of the tube body 100 and form one channel. The channel is configured to allow the transmission medium 420 to extend through. After extending through the channel, the transmission medium 420 is connected to the driving gear 410b, and each lens 200 is driven by the driven gear 410c and the lens gear 410d, and then the lens 200 can rotate synchronously in the same direction with respect to the lens body sealing head 110, such that the FOV can be extended without changing the FOV direction.

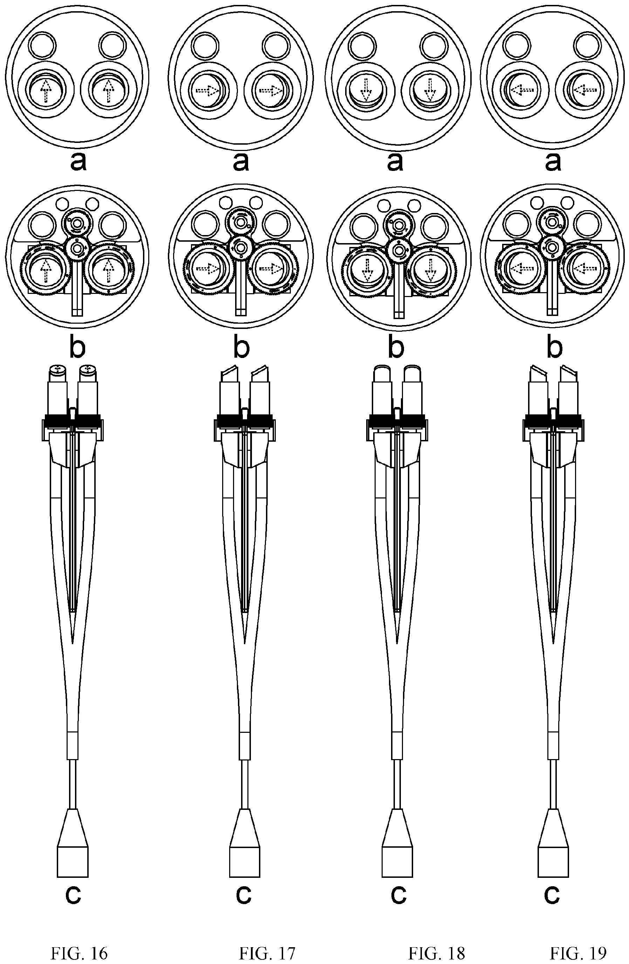

FIG. 16 to FIG. 19 are the schematic views of the electronic endoscope in the embodiment shown in FIG. 14 at four different postures of 0 °, 90 °, 180 ° and 270 ° respectively, during the process of clockwise rotation with 360 °. In the figures, *a* shows a schematic state of lens 200, *b* shows a schematic state of the first transmission device 410 corresponding to the state of *a,* and c shows the structural views of various components in the light receiving channel of the electronic endoscope in the state of *a.* It should be understood that the dashed arrows in the figures are for the convenience of explanation only and do not constitute a limitation to the present application. It can be seen from the figures that, when the driving gear 410b drives the driven gear 410c clockwise, the driven gear 410c will rotate anticlockwise, and then the two lens gears 410d are driven to rotate clockwise simultaneously, thus the two lenses 200 are driven to rotate clockwise synchronously, while the PCB 320 and the solid camera element 310 will not rotate, such that the visual line can be expanded without changing the FOV direction.

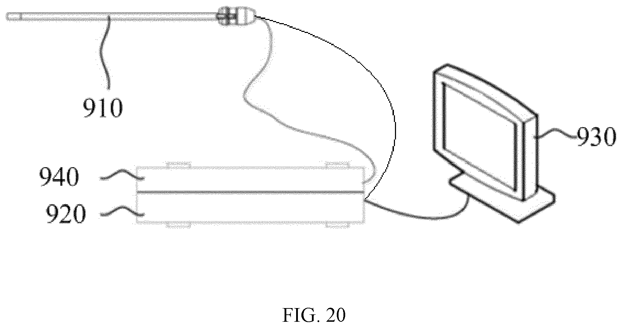

An electronic endoscope system is also provided according to an embodiment of the present disclosure, as shown in FIG. 20. The electronic endoscope system includes an electronic endoscope 910, an image workstation 920, and a monitor 930. The electronic endoscope 910 can adopt the electronic endoscope described in any of the above-mentioned embodiments. The image workstation 920 is communicatively connected to the image sensing assembly 300 in the electronic endoscope 910 and configured to receive and process the output image information. Specifically, the image workstation can process the image with algorithms, such as decode, difference, sharpen, enhance, shadow processing and white balance etc..

The monitor 930 is connected to the image workstation 920 and configured to receive and display the image processed by the image workstation 920, so as to facilitate the operator to observe. Further, the monitor 930 and the image workstation 920 can be integrated on one terminal.

Further, the lighting device 940 in the electronic endoscope system is independent provided from the electronic endoscope 910, so as to facilitate the replacement of the lighting device 940 and reduce the volume of the electronic endoscope 910.

Each technical feature of the above-mentioned embodiment can be arbitrarily combined. In order to make the description concise, all possible combinations of each technical feature in the above-mentioned embodiment are not described. However, as long as the combination of these technical features is not contradictory, it shall be considered as in the scope of the description.

The above-mentioned embodiments only express several embodiments of the application, and the description is more specific and detailed, but it cannot be understood as a restriction on the scope of patent application. It should be pointed out that for ordinary technical personnel in the art, certain modifications and improvements can be made without departing from the concept of the application, which fall within the protection scope of the application. Therefore, the protection scope of the application shall be subject to the attached claims.

## Claims

1. An electronic endoscope, comprising a tube body, at least two non-zero degree lenses, an image sensing assembly, and a transmission module;
wherein the tube body is provided with a light receiving channel therethrough, the light receiving channel is configured to accommodate the lenses, the image sensing assembly, and the transmission module sequentially;
optical axes of the lenses are parallel to an extension axis of the light receiving channel;
the image sensing assembly is configured to convert the received image transmitted from the lenses into an electrical signal and output the electrical signal; and
the transmission module is configured to drive the lenses to rotate about the optical axes in the same direction.

2. The electronic endoscope according to claim 1, wherein the electronic endoscope further comprises a driving device, and the transmission module comprises a first transmission device, a second transmission device, and a transmission medium, wherein the driving device drives the transmission medium via the second transmission device, and the transmission medium then drives the non-zero degree lenses via the first transmission device.

3. The electronic endoscope according to claim 2, wherein
the tube body comprises a lens body sealing head and a main body of the tube body, wherein the lens body sealing head is connected to a distal end of the main body of the tube body; and
the optical receiving channel comprises a lens inner cavity located at a distal end of the lens body sealing head, a first light receiving channel located at a proximal end of the lens body sealing head, and a second light receiving channel extending through the main body of the tube body, wherein the lens extends from the lens inner cavity to the first light receiving channel, the first transmission device is located in the first light receiving channel, and the sensing component is located at the proximal end of the lens body sealing head and is fixed in the second light receiving channel.

4. The electronic endoscope according to claim 2, wherein the electronic endoscope further comprises a handheld end located at a proximal end of the tube body, the driving device is located on the handheld end, the second transmission device is located at a proximal end of the light receiving channel and is connected to the driving device, and a proximal end of the transmission medium is connected to the second transmission device, the transmission medium extends along an axial direction of the tube body, and a distal end of the transmission medium is connected to the first transmission device.

5. The electronic endoscope according to claim 4, wherein the first transmission device comprises any one of a gear transmission mechanism, a belt transmission mechanism, a chain transmission mechanism, a connecting rod transmission mechanism or a wire transmission mechanism;
the transmission medium is a transmission shaft or a steel wire; and
the second transmission device comprises a gear transmission mechanism or a connecting shaft structure.

6. The electronic endoscope according to claim 4, wherein the image sensing assembly comprises a printed circuit board and at least one solid camera element provided on the printed circuit board, the fixed camera element has a photosensitive surface, wherein an imaging surface of the lens is located on the photosensitive surface of the solid camera element.

7. The electronic endoscope according to claim 6, wherein the printed circuit board and the photosensitive surface of the solid camera element are perpendicular to the optical axis, a through hole is formed on the printed circuit board, wherein the first transmission device passes through the through hole.

8. The electronic endoscope according to claim 7, wherein the first transmission device comprises a gear transmission mechanism, the gear transmission mechanism comprises a driving gear and at least two lens gears, each of the lens gears is coupled to an outside of one lens and is engaged with the driving gear, the driving gear is connected to the transmission medium, and the driving gear drives each lens gear to rotate in the same direction, such that each lens rotates in the same direction.

9. The electronic endoscope according to claim 6, wherein the printed circuit board and the photosensitive surface of the solid camera element are arranged along a direction of the optical axis; and
the electronic endoscope further includes an optical turning component configured to convert a propagation direction of an outgoing light of the lens into a direction perpendicular to the photosensitive surface.

10. The electronic endoscope according to claim 9, wherein the first transmission device comprises a gear transmission mechanism, the gear transmission mechanism comprises a gear mounting base, a driving gear, a driven gear, and at least two lens gears, each of the lens gears is coupled to an outside of one lens, the driving gear is connected to the transmission medium, the driving gear and the driven gear are both rotatably fixed on the gear mounting base, the driving gear is externally engaged with the driven gear, and the driven gear is externally engaged with each lens gear to drive each lens gear to rotate in the same direction.

11. The electronic endoscope according to claim 9, wherein the optical turning component is a turning prism or a plane mirror.

12. The electronic endoscope according to claim 9, wherein an outgoing surface of the optical turning component is attached to the photosensitive surface of the image sensing assembly.

13. The electronic endoscope according to claim 9, wherein a channel is formed on the printed circuit board allowing the transmission medium to pass through.

14. The electronic endoscope according to claim 9, wherein
the printed circuit board comprises at least two sub printed circuit boards, each of which is symmetrically arranged with respect to the extension axis of the tube body to form a channel allowing the transmission medium to pass through.

15. The electronic endoscope according to claim 3, wherein the electronic endoscope further comprises a lens shield provided at a distal end of each lens inner cavity, and the lens shield is configured to protect the lens in the corresponding lens inner cavity.

16. The electronic endoscope according to claim 3, wherein the electronic endoscope further comprises a sealing shield provided on the lens body sealing head.

17. The electronic endoscope according to claim 16, wherein the sealing shield is connected to the lens body sealing head via a snap-fit connection or a thread connection.

18. The electronic endoscope according to claim 3, wherein the electronic endoscope further comprises an optical fiber, the lens body sealing head forms an optical output channel therethrough between the lens inner cavities, the optical output channel is in communication with the second optical receiving channel, and the optical fiber extends from the second optical receiving channel to a distal end of the lens body sealing head through the optical output channel.

19. The electronic endoscope according to claim 3, wherein the electronic endoscope further comprises a cold light source and a power supply, the lens body sealing head forms a light output channel between the lens inner cavities, and the light output channel is configured to accommodate the cold light source and the power supply.

20. An electronic endoscope system, comprising:
the electronic endoscope according to any one of claims 1 to 19;
an image workstation connected to the image sensing assembly and configured to receive image information transmitted by the image sensing assembly and process the image information; and
a monitor connected to the image workstation and configured to receive and display the image information processed by the graphic workstation.
